# FASCICULE DE BREVET EUROPEEN

(11) **EP 3 256 576 B1**
(45) Date de publication et mention de la délivrance du brevet: **28.01.2026**
(21) Numéro de dépôt: 16705089.7
(22) Date de dépôt: 12.02.2016
(51) Int. Cl.: A61P 3/04, A61P 9/00, A61P 9/10, A61P 29/00, A61P 31/22, A61P 39/06, C12N 9/02

(54) **MELANGE DE SOD PURIFIEES D'ORIGINE VEGETALE**
MISCHUNG VON GEREINIGTEN SODEN PFLANZLICHEN URSPRUNGS
MIXTURE OF PURIFIED SODS OF PLANT ORIGIN

(30) Priorité: 12.02.2015 FR 1551164
(43) Date de publication de la demande: 20.12.2017
(73) Titulaire: Bionov, 13630 Eyragues (FR)
(72) Inventeur: LACAN, Dominique, 34080 Montpellier (FR)
(74) Mandataire: Regimbeau
(86) Numéro de dépôt international: PCT/EP2016/052958
(87) Numéro de publication internationale: WO 2016/128531

(56) Documents cités:
- FR-A1- 2 716 884
- GENE E LESTER ET AL: "Superoxide Dismutase Activity in Mesocarp Tissue from Divergent Cucumis melo L. Genotypes", PLANT FOODS FOR HUMAN NUTRITION, KLUWER ACADEMIC PUBLISHERS, DO, vol. 64, no. 3, 16 June 2009 (2009-06-16), pages 205 - 211, XP019733280, ISSN: 1573-9104, DOI: 10.1007/S11130-009-0124-1
- LACAN DOMINIQUE ET AL: "High levels of antioxidant enzymes correlate with delayed senescence in nonnetted muskmelon fruits", PLANTA (BERLIN), vol. 204, no. 3, March 1998 (1998-03-01), pages 377 - 382, XP002756130, ISSN: 0032-0935

## Description

L'invention est telle que définie dans les revendications 1 à 18.

La présente invention concerne un mélange de superoxyde dismutases (SOD) extrait de Cucumis melo, en particulier la variété de melon hybride F1 nommée MA 7950 ou de ses cellules cultivées in vitro, son procédé de préparation, une composition cosmétique, nutritionnelle, vétérinaire ou pharmaceutique le contenant à titre d'ingrédient actif, ainsi que ladite composition pour son utilisation en tant que produit de soin cosmétique, de complément alimentaire, de boisson ou de médicament.

L'oxygène, indispensable à notre fonctionnement, génère pourtant des formes réactives oxygénées toxiques qui ont un effet négatif sur notre organisme. Ces formes réactives oxygénées sont pour la plupart des radicaux libres comme le radical superoxyde (O₂˙⁻), le radical hydroxyle (HO˙), le monoxyde d'azote (˙NO) ou des radicaux peroxydes d'origine lipidique (L-OO˙). L'ion superoxyde est la plus abondante des espèces réactives de l'oxygène, il est directement formé à partir de l'oxygène et est à l'origine de toutes les autres espèces réactives de l'oxygène, notamment le radical hydroxyle (HO˙), qui est très agressif.

Ces radicaux libres sont des atomes ou molécules dont la configuration électronique est caractérisée par la présence d'un électron non apparié. Cette particularité les rend instables et ils peuvent rapidement, pour se stabiliser, oxyder d'autres molécules biologiques comme les acides nucléiques (ADN), les protéines enzymatiques ou les lipides membranaires, et particulièrement les acides gras polyinsaturés (AGPI).

Notre organisme agit en permanence contre la formation d'espèces réactives de l'oxygène, qui détruisent les cellules. Dans toutes les cellules, il existe de façon constitutive plusieurs lignes de défense pour détoxifier la cellule.

La première ligne de défense est assurée par une enzyme appelée la superoxyde dismutase (SOD) qui est une métallo enzyme (Mac Cord et Fridovich, J. Biol. Chem. 244 : 6049-55, 1969). Dans le règne végétal, Il existe trois formes de SOD (Michalski, J. Chromatogr. B 684: 59-75 1996), chacune caractérisée par la présence d'un ion métallique situé au niveau de son site actif :
- une forme de SOD contient du cuivre et du zinc (Cu/Zn-SOD), localisée principalement dans le cytoplasme et le chloroplaste ;
- une autre forme contient du manganèse (Mn-SOD), elle est localisée dans la mitochondrie et le peroxysome ;
- et enfin, dans certaines espèces végétales une SOD contient du fer (Fe-SOD), et est localisée dans le chloroplaste.

Les SOD jouent un rôle clé dans la lutte contre les radicaux libres puisqu'elles permettent l'élimination de l'ion superoxyde. Les superoxydes dismutases sont des enzymes capables d'induire la dismutation des ions superoxydes, suivant la réaction :

2 O_{2˙}⁻ + 2 H₂O → O₂ + H₂O₂ + 2 OH⁻ (équation 1).

L'action de la SOD est complétée par une deuxième ligne de défense qui élimine H₂O₂ : la catalase et/ou la glutathion péroxydase-selenium dépendante (SeGPx), qui assurent la destruction du peroxyde d'hydrogène H₂O₂.

Dans des conditions biologiques normales, l'organisme humain produit constamment mais en faibles quantités des radicaux libres qui sont immédiatement neutralisés par les systèmes de défense existant. Dans certains cas, si la production de radicaux libres augmente (tabac, stress, pollution, radiations solaires, alimentation déséquilibrée...), et/ou s'il existe un déficit en substances antioxydantes, cela conduit à un déséquilibre oxydant/antioxydant, qui induit des modifications cellulaires importantes au niveau des macromolécules (oxydation de l'ADN, des protéines, des sucres, des lipides) mais aussi au niveau des organites cellulaires, en particulier les mitochondries, principales pourvoyeuses d'énergie pour la cellules qui, en cas de détérioration, deviennent une source importante de radicaux libres. Ce déséquilibre est de plus en plus étroitement corrélé à de nombreuses pathologies et de nombreux déséquilibres. La SOD, en neutralisant l'anion superoxyde, à l'origine de toutes les espèces réactives de l'oxygène et donc du stress oxydant, joue un rôle clé dans la régulation du stress oxydant et l'administration de SOD exogène est envisagée dans le traitement des pathologies liées au stress oxydant.

Jusqu'à présent, les seules SOD purifiées disponibles étaient issues du règne animal, notamment extraites d'érythrocytes de bœuf (Markovitz, J. Biol. Chem., 234, p. 40, 1959*),* d'*Escherichia Coli* (Keele et Fridovitch, J. Biol., 245, p. 6176, 1970) et de souches bactériennes marines (brevets FR 2 225 443 et FR 2 240 277).

En effet, la SOD, principalement la forme Cu-Zn-SOD bovine, a fait l'objet d'un développement pharmaceutique qui a conduit à un médicament appelé Orgotein^{®}. Elle a été utilisée dans des pathologies induites par des radicaux libres, notamment dans le cas d'inflammations chroniques comme par exemple dans le traitement de la maladie de Crohn (Emerit et al., 1991, Free Rad. Res. Comms, 12-13, 563-569) ou encore dans le cas de fibroses radio-induites (Delanian et al. 1994, Radiotherapy and Oncology, 32, 12-20). Néanmoins, pour des raisons liées notamment aux substances infectieuses que peuvent contenir ces matières, ces SOD animales ont, au moins en France, rapidement été interdites dans les années 1990. De ce fait, la recherche d'une source de SOD issue du règne végétal fait l'objet d'un intérêt constant dans le domaine de la pharmacie.

Des compositions alternatives comprenant de la SOD d'origine végétale, et notamment de melon, ont donc été proposées. Les bénéfices tirés de l'utilisation de compléments alimentaires à base de melon sont en effet bien connus, comme l'illustre l'article de Milind et al (International Research Journal of Pharmacy, 2011, 8, 52-57), l'article Voudoukis et Lacan et al (Journal of Ethnopharmacology 2004, 94, 67-75), ou l'article de Gene et al (Journal of Agricultural and Food Chemistry, 2008, 56, 3694-3698).

En outre, les melons présentent en général une teneur particulièrement élevée en SOD par rapport aux autres fruits. Le rendement d'extraction de SOD à partir de melons est alors élevé et rentable économiquement. Il est par ailleurs à noter que des extraits issus d'autres fruits, contenant une teneur suffisamment élevée pour observer un effet dû à la présence de SOD, ne sont pas à ce jour disponibles dans le commerce.

Ainsi, des formulations de SOD d'origine végétale, et en particulier de melon, enrobées dans une matrice de gliadine de blé ont également été décrites, par exemple dans la demande de brevet FR 2 729 296 ou pour le produit GLISODIN^{®}, dans le but de protéger des sucs gastriques les compositions comprenant de la SOD destinées à une administration par voie orale.

Par ailleurs, le brevet FR 2 716 884 décrit un extrait protéique de Cucumis melo, de préférence la variété 95LS444, présentant une activité enzymatique superoxyde dismutase supérieure à 30 unités/mg de protéines solubles, leur procédé de préparation et leur utilisation dans des compositions pharmaceutiques ou cosmétiques à usage topique externe. Toutefois, un tel extrait protéique, bien que riche en superoxyde dismutase, ne peut pas être utilisé pour de nombreuses applications pharmaceutiques, notamment car il n'est pas assez purifié, ni assez actif. En effet, il est indiqué dans l'exemple 1 que l'extrait protéique de Cucumis Melo de variété 95LS444 présente une activité enzymatique superoxyde dismutase de 126 U/mg protéines. Un tel extrait, issu d'un melon Clipper (descendant de la variété Cucumis Melo de variété 95LS444) est également décrit, avec la même teneur globale en SOD dans Lacan et al (Planta 1998, 204, 377-382). On notera que cet article décrit un unique isoforme de Fe-SOD.

Il existe donc un besoin pour l'obtention d'un mélange de SOD d'origine végétale, avec une activité pharmaceutique améliorée, pour lutter contre les maladies liées au stress oxydant et leurs conséquences, en particulier contre les maladies cardiovasculaires et l'obésité et leurs conséquences.

De manière surprenante, le demandeur a trouvé un mélange de SOD d'origine végétale qui possède des propriétés anti-oxydantes supérieures aux mélanges de SOD de l'art antérieur. En particulier, le mélange de SOD de l'invention est plus actif qu'un mélange de SOD issu d'un extrait protéique de Cucumis Melo de variété 95LS444 ou d'un de ses descendants, tel le melon Clipper.

Sans pour autant être limité à cette interprétation, il semble que la présence dans ledit mélange d'une isoforme particulière de superoxyde dismutase au fer, qui n'a jamais été décrite à ce jour, puisse conférer au mélange selon l'invention ses propriétés pharmaceutiques supérieures.

La présente invention concerne donc en premier lieu un mélange de superoxyde dismutases d'origine végétale constitué essentiellement de 3 superoxyde dismutases tel que défini dans la revendication 1.

Le mélange de SODs selon l'invention possède des propriétés surprenantes et des effets bénéfiques sur le système cardiovasculaire, notamment sur l'hypertrophie cardiaque. En effet, le mélange de SODs selon l'invention module de façon surprenante l'expression de certains gènes associés aux pathologies cardiaques et à l'obésité comme le gène NPPA ou RXFP1.

Les modes de réalisation suivants ne sont pas couverts par le texte des revendications mais sont considérés comme utiles à la compréhension de l'invention :
- une isoforme de SOD au Fer (Fe-SOD) d'origine végétale présentant une activité antioxydante particulièrement intéressante ;
- un procédé de préparation dudit mélange de superoxyde dismutases d'origine végétale constitué essentiellement de 3 superoxyde dismutases ;
- un procédé de préparation de ladite isoforme de SOD au Fer (Fe-SOD) d'origine végétale présentant une activité antioxydante particulièrement intéressante.

La présente invention concerne également une composition cosmétique nutritionnelle, vétérinaire ou pharmaceutique contenant à titre d'ingrédient actif un mélange de superoxyde dismutases purifiées selon l'invention ou l'isoforme de SOD au Fer (Fe-SOD) d'origine végétale selon l'invention, et au moins un excipient alimentaire, pharmaceutiquement ou cosmétiquement acceptable.

La présente invention concerne également une composition selon l'invention pour son utilisation en tant que médicament.

La présente invention concerne donc en premier lieu un mélange original de superoxyde dismutases d'origine végétale constitué essentiellement de 3 superoxyde dismutases tel que défini dans la revendication 1 : une superoxyde dismutase au manganèse, une superoxyde dismutase au cuivre et au zinc et une superoxyde dismutase au fer présente sous au moins deux isoformes, la première isoforme de superoxyde dismutase au fer avec un poids moléculaire compris entre 28 000 et 36 000 Da, la deuxième isoforme de superoxyde dismutase au fer avec un poids moléculaire compris entre 75 000 et 85 000 Da, ledit mélange étant susceptible d'être obtenu à partir d'un extrait de la variété hybride F1 de Cucumis Melo MA 7950 ou de ses cellules cultivées in vitro, et ledit mélange présentant de préférence une activité SOD totale supérieure ou égale à 130 U/mg du mélange.

Ledit mélange selon l'invention est susceptible d'être obtenu à partir d'un extrait de la variété hybride F1 de Cucumis Melo MA 7950 ou de ses cellules cultivées in vitro, de manière encore préférée, ledit mélange est susceptible d'être obtenu à partir d'un extrait de la variété hybride F1 de Cucumis Melo MA 7950. On note incidemment que les Cucumis Melo descendant de la lignée cellulaire MA 7950 ou de l'une des variétés hybrides issues de MA 7950 présentent une teneur particulièrement élevée en SOD, à la fois par rapport aux autres fruits et par rapport aux autres variétés de melons. Ainsi, le rendement d'extraction de la SOD à partir de ces Cucumis Melo spécifiques est particulièrement élevé et rentable économiquement.

Par « constitué essentiellement de », on entend au sens de la présente invention que le mélange comprend entre 70 et 99.9%, avantageusement de 80 à 99.9%, en poids de superoxyde dismutases. Les autres constituants du mélange présentent une incidence minime sur son activité enzymatique. De préférence, les autres constituants n'interfèrent pas avec l'activité enzymatique superoxyde dismutase.

De préférence, le mélange selon l'invention est extrait de la variété hybride F1 de Cucumis Melo MA 7950, ou de ses cellules cultivées in vitro.

On notera que l'homme du métier, à l'aide de ses connaissances générales, peut obtenir la séquence peptidique des SOD du mélange selon l'invention, puis à l'aide de techniques bien connues, peut générer l'ARNm (ARN messager) et l'ADNc (ADN complémentaire) correspondant. Ce dernier pourra être transféré à l'aide de techniques bien connues dans des cellules procaryotes ou eucaryotes, qui serviront ensuite à produire le mélange de SOD selon l'invention. Ce mode de réalisation ne relève pas de l'invention est n'est présent qu'à titre illustratif.

Les « cellules de Cucumis Melo MA 7950 cultivées in vitro » comprennent, au sens de la présente invention, aussi bien les cellules directement issues de la plante et cultivées in vitro selon des techniques bien connues de l'homme du métier, et notamment les cellules souches, que des cellules issues de ces premières cellules.

La variété hybride F1 de Cucumis melo MA 7950, dont les semences ont été déposées conformément au Traité de Budapest dans la collection NCIMB (National Collection of Industrial and Marine Bacteria-ABERDEEN AB2 IRY (Ecosse - GB) 23 St. Machar Drive) le 8 juillet 2013 sous le numéro NCIMB 42154, possède des caractéristiques uniques quant à son aspect, sa résistance au stress et sa composition en SOD. Ce mélange de SOD possède des propriétés anti-oxydantes supérieures à d'autres mélanges de SOD issues d'autres sources végétales, et notamment issues d'autres variétés de melon.

Par « extrait », on entend au sens de la présente invention un extrait protéique, de préférence un extrait protéique soluble.

Par « SOD », on entend au sens de la présente invention une enzyme de type superoxyde dismutase. Il est à noter que les superoxyde dismutases selon l'invention sont naturelles, c'est-à-dire qu'elles ne sont pas modifiées de manière chimique. En particulier, la présente invention vise les SOD dans leur intégrité, et non des fragments de celles-ci. Les SOD sont classées en trois catégories, en fonction du métal présent dans leur site actif : les superoxyde dismutases au manganèse (Mn-SOD), les superoxyde dismutases au cuivre et au zinc (Cu/Zn-SOD) et les superoxyde dismutases au fer (Fe-SOD).

Dans le mélange selon la présente invention, on trouve au moins deux isoformes de Fe-SOD telles que définies dans la revendication 1. Les isoformes d'une protéine sont les différentes formes qu'elle prend lorsqu'elle est issue de gènes différents, ou du même gène par épissage alternatif. Dans ce qui suit, on nommera ces deux isoformes de Fe-SOD « première isoforme » et « deuxième isoforme » en fonction de leur poids moléculaire. Ainsi, la « première isoforme » selon l'invention présente un poids moléculaire plus petit que celui de la « deuxième isoforme ».

Il est à noter que les autres SOD (Mn-SOD et/ou Cu/Zn-SOD) peuvent également être présentes dans le mélange sous plusieurs isoformes. Ainsi, selon la présente invention, le terme Mn-SOD (respectivement Cu/Zn-SOD) recouvre l'ensemble des isoformes de Mn-SOD (respectivement Cu/Zn-SOD) présentes dans le mélange.

Les SOD catalysent la dismutation des ions superoxydes suivant la réaction :

2 O_{2˙}⁻ + 2 H₂O → O₂ + H₂O₂ + 2 OH⁻ (équation 1).

Le mélange selon l'invention est caractérisé par son activité SOD totale. Par « activité SOD totale» on entend au sens de la présente invention la quantification de la réaction de dismutation décrite dans l'équation 1 par le mélange selon l'invention. Cette activité SOD totale est mesurée selon des techniques bien connues de l'homme du métier et est exprimée en U (unité enzymatique ou unité) par mg de protéines. De préférence, elle est mesurée selon une méthode basée sur la réduction du sel de tétrazolium. On peut notamment citer la méthode de Beauchamp et Fridovich (Anal. Biochem. 44 :276-82 (1971)) basée sur l'inhibition de la réduction du Nitroblue Tetrazolium (N 55 14 Sigma-Aldrich, France) par la SOD, et modifiée par Oberley et Spitz en 1985 (Boca Raton CRC Press; In R.A. Grenwald 1985, ed: Handbook of Methods for oxygen radical research, p217-230). On peut également utiliser le kit d'analyse SOD assay kit (19160, Sigma-Aldrich, France), basé également sur la réduction du sel de tétrazolium, qui est une variante de la méthode précédente mais plus facile à mettre en œuvre.

Le mélange de SOD selon l'invention présente de préférence une activité SOD totale supérieure à 130 U/mg dudit mélange, de manière encore plus préférée, supérieure à 300 U/mg dudit mélange, de manière préférée entre toutes supérieure à 500 U/mg dudit mélange.

Par ailleurs, il est également possible de déterminer l'activité SOD attribuée à chaque SOD (Mn-SOD, Cu/Zn-SOD et la Fe-SOD). Cette activité SOD « relative » quantifie la contribution de chaque SOD (ou d'une de ses isoformes) à l'activité SOD totale du mélange. Elle est ainsi exprimée en pourcentage de l'activité SOD totale du mélange. L'activité SOD de chaque SOD (ou de l'une de ses isoformes) est mesurée à l'aide de techniques bien connue de l'homme du métier. En effet, les différentes formes de SOD peuvent être identifiées en utilisant différents inhibiteurs : KCN inhibe l'activité de la Cu/Zn SOD ; H₂O₂ inhibe l'activité de la Fe SOD et de la Cu/Zn SOD alors que la Mn SOD est insensible à H₂O₂ et KCN (Paul et Van Alstyne, J Exp. Mar. Biol. Ecol. 160 : 191-203 1992).

Ainsi, on mesure tout d'abord l'activité SOD totale du mélange.

La méthode de détermination des différentes formes de SOD, bien connue de l'homme du métier, consiste en une séparation par électrophorèse en gel d'acrylamide dans des conditions natives, suivie par une détection de l'activité SOD sur le gel.

Des échantillons du mélange de SOD selon l'invention sont déposés dans les différents puits d'un gel d'électrophorèse. La migration en conditions non dénaturantes est ensuite effectuée. Lors de l'étape de révélation du gel, celui-ci est découpé en 3 parties. La première est révélée en activité SOD totale, la deuxième est préincubée avec 2 mM de KCN puis révélée en activité SOD dans les mêmes conditions, enfin la dernière est préincubée avec 5 mM de H₂O₂ puis révélée en activité SOD dans les mêmes conditions. Les intensités des différentes bandes sont mesurées par intégration de l'air sous la courbe, et leur rapport indique les activités SOD « relative » des différentes SOD (ou de leurs isoformes) du mélange selon l'invention. Un exemple de détermination de l'activité SOD « relative » des différentes SOD du mélange est illustré dans les exemples.

Ainsi, le mélange de superoxyde dismutases selon l'invention est tel que l'activité SOD cumulée des deux isoformes de superoxyde dismutases au fer est comprise entre 20% et 26%, avantageusement entre 22% et 26% de l'activité SOD totale du mélange.

Selon une variante préférée de l'invention, le mélange de superoxyde dismutases purifiées est tel que l'activité SOD cumulée des deux isoformes de superoxyde dismutase au fer est comprise entre 20% et 26%, avantageusement égale à 25%, de l'activité SOD totale du mélange, l'activité de la superoxyde dismutase au cuivre et au zinc est comprise entre 60% et 70%, avantageusement égale à 65%, de l'activité SOD totale du mélange, et l'activité de la superoxyde dismutase au manganèse est comprise entre 9 et 15%, avantageusement comprise entre 7 et 12%, encore plus avantageusement égale à 10% de l'activité SOD totale du mélange.

Les SOD, comme toutes les protéines, peuvent être séparées en fonction de deux caractéristiques biochimiques : leur masse moléculaire et leur point isoélectrique.

La séparation des différentes formes de SOD peut être effectuée par séparation par électrophorèse en gel d'acrylamide dans des conditions natives, suivie par une détection de l'activité SOD sur le gel. La visualisation des différentes SOD et la mesure de l'activité SOD sont réalisées grâce à la méthode de Beauchamp et Fridovich Anal. Biochem. 44 :276-82 (1971).

Les poids moléculaires des différentes formes de SOD peuvent être déterminés en utilisant des marqueurs de poids moléculaires (protéines de poids moléculaire connu) pour étalonner le gel d'électrophorèse. Les différentes formes de SOD sont révélées comme décrit ci-dessus et la position des bandes obtenues est comparée à celles des marqueurs de poids moléculaire afin de déterminer les poids moléculaires de chaque isoforme de SOD.

Ainsi, le mélange selon l'invention comprend une superoxyde dismutase au fer présente sous au moins deux isoformes, la première avec un poids moléculaire compris entre 28 000 et 36 000 Da, la deuxième avec un poids moléculaire compris entre 75 000 et 85 000 Da.

Avantageusement, la première isoforme de superoxyde dismutase au fer du mélange de SOD selon l'invention présente un poids moléculaire d'environ 32 200 Da.

Avantageusement, la deuxième isoforme de superoxyde dismutase au fer du mélange de SOD selon l'invention présente un poids moléculaire d'environ 79 800 Da.

Dans un mode de réalisation préféré, la superoxyde dismutase au manganèse présente un poids moléculaire compris entre 70 000 et 90 000 Da et la superoxyde dismutase au cuivre et au zinc présente un poids moléculaire compris entre 27 000 et 35 000 Da.

Selon une variante préférée de l'invention, la Cu/Zn-SOD a un poids moléculaire compris entre 27 000 et 35000 Da, avantageusement égal à 31 800 Da, la Mn-SOD un poids moléculaire compris entre 70 000 et 90 000 Da, avantageusement égal à 80 6000 Da. Pour les Fe-SOD, la première isoforme présente un poids moléculaire compris entre 28 000 et 36 000 Da, avantageusement égal à 32 200 Da, la deuxième isoforme Fe-SOD présente un poids moléculaire compris entre 75 000 et 85 000 Da, avantageusement égal à 79 800 Da.

La séparation des SOD en fonction de leur charge se fait par isoélectrofocalisation (IEF). Les SOD migrent dans un champ électrique et s'immobilisent quand l'environnement de pH rend leur charge globale nulle. Les points isoélectriques (ou pHi) des différentes formes de SOD peuvent être déterminés sur les gels d'IEF par comparaison à des marqueurs de point isoélectrique connu (IEF markers 3.6-9.3, Référence 56733, Sigma-Aldrich, France).

Par point isoélectrique ou pHi, on entend au sens de la présente invention, le pH pour lequel la charge globale de cette molécule est nulle ou, autrement dit, le pH pour lequel la molécule est électriquement neutre.

De préférence, la Cu/Zn-SOD a un pHi de 4.3.

Avantageusement, les deux isoformes (la première et la deuxième) de la Fe-SOD ont un pHi de 4.4 et 4.7 respectivement.

Selon une variante préférée de l'invention, la Cu/Zn-SOD a un pHi de 4.3, les Fe-SOD ont un pHi de 4.4 et 4.7 et les différentes isoformes de la Mn SOD ont des pHi de 4.1 ; 4.4 ; 5.3 ; 5.5 ; 5.7 ; 5 .85 ; 6.1.

Les modes de réalisation suivants ne sont pas couverts par le texte des revendications mais sont considérés comme utiles à la compréhension de l'invention. En particulier, le procédé de préparation du mélange de superoxyde dismutase selon l'invention comprend les étapes successives de :
- broyage ou pressage en milieu aqueux, de préférence à pH de 5 à 9, de la variété hybride F1 de Cucumis Melo MA 7950 ou de ses cellules cultivées in vitro,
- récupération du surnageant, et
- purification par chromatographie, en particulier par chromatographie IMAC.

Ainsi, le mélange de superoxyde dismutases selon l'invention, de préférence purifiées, est susceptible d'être obtenu par le procédé comprenant les étapes successives de :
- broyage ou pressage en milieu aqueux, de préférence à pH de 5 à 9, de la variété hybride F1 de Cucumis Melo MA 7950 ou de ses cellules cultivées in vitro,
- récupération du surnageant, et
- purification par chromatographie, en particulier par chromatographie IMAC (« Immobilized metal ion affinity chromatography »).

La chromatographie d'affinité sur ions métalliques immobilisés (IMAC) est basée sur l'affinité de certains acides aminés, en particulier l'histidine le tryptophane et la cystéine, pour les métaux. Ainsi, cette technique chromatographique fonctionne en permettant à des protéines ayant une affinité pour les ions métalliques d'être retenues sur une colonne contenant des ions métalliques immobilisés, tels que le cobalt, le nickel, le cuivre. Les éluants utilisés présentent en général un gradient de pH, ou de concentration d'une molécule compétitive pour se lier aux ions de la colonne, tels que l'imidazole. De manière préférée, on utilisera la chromatographie IMAC en utilisant une colonne comprenant des ions cuivre en tant qu'ions métalliques immobilisés. Ainsi, la vitesse d'élution des protéines est dépendante de leur charge et de leur affinité à l'ion métallique, en particulier le cuivre, et est donc corrélée à la présence d'acides aminés comme le tryptophane, l'histidine, la cystéine dans la séquence protéique. Avantageusement, on utilisera en tant qu'éluant un gradient de solutions aqueuses de NH₄Cl et CuSO₄.

Alternativement, le procédé selon la description comprend les étapes successives de :
- broyage ou pressage en milieu aqueux, de préférence à pH de 5 à 9, de la variété hybride F1 de Cucumis Melo MA 7950 ou de ses cellules cultivées in vitro,
- récupération du surnageant, et
- purifications membranaires successives, en particulier par passage du surnageant à travers des membranes à porosité variable.

L'homme du métier saura adapter le gradient de porosité des membranes dans le procédé en fonction de la maturité et du taux de matière sèche du melon, en particulier en cherchant à éviter le colmatage des membranes.

Comme indiqué plus haut, il semble que la présence de la deuxième isoforme de la superoxyde dismutase au fer, qui n'a jamais été décrite à ce jour, puisse conférer au mélange selon l'invention ses propriétés biologiques supérieures.

Cette deuxième isoforme de la superoxyde dismutase au fer susceptible d'être obtenu à partir d'un extrait de la variété hybride F1 de Cucumis Melo MA 7950 présente un poids moléculaire compris entre 75 000 et 85 000 Da.

Ainsi, plus généralement, la présente description concerne une superoxyde dismutase SODᵢ (synthétique ou naturelle) dont la séquence polypeptidique est homologue à la séquence de la deuxième isoforme, et présente en particulier une identité de séquence supérieure ou égale à 80%, de préférence 85%, 90%, 95% ou 98%, avec la séquence de la deuxième isoforme.

Une séquence polypeptidique homologue de la deuxième isoforme inclut toute séquence polypeptidique qui diffère de la séquence de la deuxième isoforme par mutation, insertion, délétion ou substitution d'une ou plusieurs acides aminés, pour autant qu'elle présente l'activité biologique de la deuxième isoforme.

Par « pourcentage d'identité » entre deux séquences d'acides aminés au sens de la présente invention, on entend désigner un pourcentage de résidus d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Les comparaisons de séquences entre deux séquences d'acides aminés sont traditionnellement réalisées en comparant ces séquences après les avoir alignées de manière optimale, ladite comparaison pouvant être réalisée par segment ou par « fenêtre de comparaison ». L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acides aminés est déterminé en comparant ces deux séquences alignées de manière optimale dans laquelle la séquence d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 sequences » (Tatusova et al., « Blast 2 sequences - a new tool for comparing protein and nucleotide sequences », FEMS Microbiol., 1999 Lett. 174:247-250) disponible sur le site http://www.ncbi.nlm.nih.gov/gorf/bl2.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

On entend par « acide aminé », au sens de la présente invention, tous les résidus des acides α-aminés naturels (par exemple Alanine (Ala), Arginine (Arg), Asparagine (Asn), Acide aspartique (Asp), Cystéine (Cys), Glutamine (Gln), Acide glutamique (Glu), Glycine (Gly), Histidine (His), Isoleucine (Ile), Leucine (Leu), Lysine (Lys), Méthionine (Met), Phénylalanine (Phe), Proline (Pro), Sérine (Ser), Thréonine (Thr), Tryptophane (Trp), Tyrosine (Tyr) et Valine (Val)) sous la forme D ou L, ainsi que les acides aminés non naturels (par exemple, la β-alanine, l'allylglycine, la tert-leucine, l'acide 3-amino-adipique, l'acide 2-aminobenzoïque, l'acide 3-aminobenzoïque, l'acide 4-aminobenzoïque, l'acide 2-aminobutanoïque, la 4-amino-1-carboxyméthyl pipéridine, l'acide 1-amino-1-cyclobutanecarboxylique, l'acide 4-aminocyclohexaneacétique, l'acide 1-amino-1-cyclohexanecarboxyilique, l'acide (1R,2R)-2-aminocyclohexanecarboxylique, l'acide (1R,2S)-2-aminocyclohexanecarboxylique, l'acide (1S,2R)-2-aminocyclohexanecarboxylique, l'acide (1S,2S)-2-aminocyclohexanecarboxylique, l'acide 3-aminocyclohexanecarboxylique, l'acide 4-aminocyclohexanecarboxylique, l'acide (1R,2R)-2-aminocyclopentanecarboxylique, l'acide (lR,2S)-2-aminocyclopentanecarboxyilique l'acide 1-amino-1-cyclopentanecarboxylique, l'acide 1-amino-1-cyclopropanecarboxylique, l'acide 4-(2-aminoéthoxy)-benzoïque, l'acide 3-aminométhylbenzoïque, l'acide 4-aminométhylbenzoïque, l'acide 2-aminobutanoïque, l'acide 4-aminobutanoïque, l'acide 6-aminohexanoïque, l'acide 1-aminoindane-1-carboxylique, l'acide 4-aminométhyl-phénylacétique, l'acide 4-aminophénylacétique, l'acide 3-amino-2-naphtoïque, l'acide 4-aminophénylbutanoïque, l'acide 4-amino-5-(3-indolyl)-pentanoïque, l'acide (4R,5S)-4-amino-5-méthylheptanoïque, l'acide (R)-4-amino-5-méthylhexanoïque, l'acide (R)-4-amino-6-méthylthiohexanoïque, l'acide (S)-4-amino-pentanoïque, l'acide (R)-4-amino-5-phénylpentanoïque, l'acide 4-aminophénylpropionique, l'acide (R)-4-aminopimérique, l'acide (4R,5R)-4-amino-5-hyroxyhexanoïque, l'acide (R)-4-amino-5-hydroxypentanoïque, l'acide (R)-4-amino-5-(p-hydroxyphényl)-pentanoïque, l'acide 8-aminooctanoïque, l'acide (2S,4R)-4-amino-pyrrolidine-2-carboxylique, l'acide (2S,4S)-4-amino-pyrrolidine-2-carboxylique, l'acide azétidine-2-carboxylique, l'acide (2S,4R)-4-benzyl-pyrrolidine-2-carboxylique, l'acide (S)-4,8-diaminooctanoïque, l'acide tert-butylglycine, le γ-carboxyglutamate, la β-cyclohexylalanine, la citruline, l'acide 2,3-diamino propionique, l'acide hippurique, l'homocyclohexylalanine, la moleucine, l'homophénylalanine, la 4-hydroxyproline, l'acide indoline-2-carboxylique, l'acide isonipécotique, l'α-méthyl-alanine, l'acide nicopetique, la norleucine, la norvaline, l'acide octahydroindole-2-carboxylique, l'ornithine, la pénicillamine, la phénylglycine, l'acide 4-phényl-pyrrolidine-2-carboxylique, l'acide pipécolique, la propargylglycine, la 3-pyridinylalanine, la 4-pyridinylalanine, l'acide 1-pyrrolidine-3-carboxylique, la sarcosine, les statines, l'acide tétrahydroisoquinoline-1-carboxylique, l'acide 1,2,3,4-tétrahydroisoquinoline-3-carboxylique, l'acide tranexamique). Le terme comprend aussi les acides aminés naturels et non naturels portant un groupe amino-protecteur conventionnel (par exemple un groupe acétyle, tert-butyloxycarbonyle, benzyloxycarbonyle ou 9-fluorénylméthylcarbonyle), ainsi que les acides aminés naturels et non naturels protégés à l'extrémité carboxylique (avantageusement par un groupe alkyle en (C1-C18), un ester, une amide phénylique ou benzylique ou une amide, ce qui donne respectivement des extrémité carboxylique de formule suivante :-CO(alkyle en C1-C18), -COO(alkyle en C1-C18), -CONHphényle CONHbenzyle ou CONH2).

Par séquence d'acide aminé présentant au moins 80 %, de préférence 85 %, 90 %, 95 % et 98 % d'identité avec une séquence d'acide aminé de référence, on préfère celles présentant par rapport à la séquence de référence, certaines modifications, en particulier une délétion, addition ou substitution d'au moins un acide aminé, une troncation ou un allongement. Dans le cas d'une substitution, d'un ou plusieurs acide(s) aminé(s) consécutif(s) ou non consécutif(s), on préfère les substitutions dans lesquelles les acides aminés substitués sont remplacés par des acides aminés « équivalents ». L'expression « acides aminés équivalents » vise ici à désigner tout acide aminé susceptible d'être substitué à l'un des acides aminés de la structure de base sans cependant modifier essentiellement les activités biologiques des anticorps correspondants et telles qu'elles seront définies par la suite, notamment dans les exemples.

Ces acides aminés équivalents peuvent être déterminés en s'appuyant par exemple sur leur homologie de structure avec les acides aminés auxquels ils se substituent.

Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales polaires (tels que la glycine, l'alanine, la valine, la leucine, l'isoleucine, la proline, la phénylalanine, la méthionine, le tryptophane, et la cystéine).

De préférence, les superoxyde dismutases « variantes », « homologues », ou « dérivées » sont de même longueur que les séquences de référence. Ainsi, de manière avantageuse, la séquence de la superoxyde dismutase selon l'invention SODᵢ est constituée de la séquence de la deuxième isoforme de la superoxyde dismutase au fer susceptible d'être obtenu à partir d'un extrait de la variété hybride F1 de Cucumis Melo MA 7950 avec un poids moléculaire compris entre 75 000 et 85 000 Da.

La deuxième isoforme de la superoxyde dismutase au fer est susceptible d'être obtenue à partir d'un extrait de la variété hybride F1 de Cucumis Melo MA 7950 avec un poids moléculaire compris entre 75 000 et 85 000 Da. Celle-ci peut être obtenue selon un procédé comprenant les étapes successives de :
- broyage ou pressage en milieu aqueux à pH de 5 à 9 de la variété hybride F1 de Cucumis Melo MA 7950 ou de ses cellules cultivées in vitro,
- récupération du surnageant, et
- purification par chromatographie, en particulier par chromatographie IMAC.

La présente description vise également tout mélange comprenant ladite superoxyde dismutase SODᵢ.

La présente invention concerne également une composition cosmétique, nutritionnelle, vétérinaire ou pharmaceutique contenant à titre d'ingrédient actif le mélange de superoxyde dismutases selon l'invention, et au moins un excipient alimentaire ou pharmaceutiquement ou cosmétiquement acceptable.

Au sens de la présente invention, le terme « composition pharmaceutique » recouvre aussi bien une composition à usage pharmaceutique ou vétérinaire, c'est-à-dire qu'il est destiné à être utilisé pour traiter un animal, y compris un homme.

Par « animal », on entend au sens de la présente invention par exemple un mammifère, un poisson, un crustacé, un reptile, un oiseau. En particulier, le mammifère comprend les hommes.

Au sens de la présente invention, le terme « composition nutritionnelle » englobe notamment les compositions nutraceutiques (en particulier les compléments alimentaires par exemple sous forme solide ou liquide), les compositions diététiques et les boissons, notamment à caractère diététique ou nutritionnel telles que les boissons aux vertus antioxydantes. De préférence, une composition nutritionnelle comprend les compositions nutraceutiques et les boissons diététiques telles que les boissons aux vertus antioxydantes. Dans la présente description, on entend désigner par excipient alimentaire ou pharmaceutiquement ou cosmétiquement acceptable, un composé ou une combinaison de composés entrant dans une composition nutritionnelle, pharmaceutique ou cosmétique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces excipients acceptables sont bien connus et seront adaptés par l'homme de l'art en fonction de la nature et du mode d'administration du ou des composés actifs choisis.

Avantageusement, la composition nutritionnelle, cosmétique ou pharmaceutique selon l'invention présente une teneur en superoxyde dismutase(s) comprise entre 0,01 et 10% en poids, par exemple entre 0,01 et 5 % en poids de superoxyde dismutase(s), ou encore entre 0,01 et 1 % en poids, par rapport au poids total de la composition.

Dans un mode de réalisation, la composition cosmétique selon l'invention est destinée à un usage topique externe telle que les produits de soins, les shampoings, les lotions, les gels.

Lorsque la composition selon l'invention est destinée à un usage nutritionnel, celle-ci est avantageusement destinée à une administration par voie orale. Par exemple, la composition nutritionnelle selon l'invention se présente sous forme de comprimé, de gélule, de capsule molle (soft gel), de comprimé effervescent, de sachet ou de stick à diluer, de gommes à mâcher, de boissons, de jus, de yaourt, de confiserie, de biscuit ou de barres.

Lorsque la composition selon l'invention est destinée à un usage pharmaceutique, celle-ci est destinée à une administration par voie topique, orale, nasale ou parentérale. Par exemple, la composition pharmaceutique selon l'invention se présente sous forme de comprimé, de gélule, de capsule molle (soft gel), de comprimé effervescent, de sachet ou de stick à diluer, de sirop, d'élixir, de tisanes, de gommes à mâcher, de spray, d'aérosol ou de solution injectable.

Dans un mode de réalisation préféré, la composition pharmaceutique selon l'invention est destinée à une administration par voie orale.

Pour la voie orale, la composition selon l'invention peut être protégée efficacement des sucs gastriques par des enrobages bien connus de l'homme de l'art, notamment des matières grasses végétales voir (brevet FR 2 822 381) ou dans des particules d'amidon modifié (voir demande internationale WO2006/030111).

Ainsi les compositions selon l'invention peuvent être délivrées efficacement par voie orale et induisent une augmentation de la synthèse de superoxyde dismutase et de glutathion peroxydase endogènes dans chaque organe où un stress oxydant est présent, ce qui permet une lutte efficace et constante contre les pathologies liées au stress oxydant. Dans un mode de réalisation particulier, la composition pharmaceutique, cosmétique, vétérinaire ou nutritionnelle selon l'invention contient un autre principe actif, avantageusement un autre antioxydant.

La présente invention concerne également une composition selon l'invention pour son utilisation en tant que médicament.

L'administration du mélange de SOD selon l'invention induit la synthèse endogène, par la cellule, de SOD et d'une enzyme de la deuxième ligne de défense: la glutathion peroxydase. Ceci permet à la cellule et à l'organisme de lutter efficacement et durablement contre le stress oxydant.

Ainsi, le médicament est destiné de préférence à traiter ou prévenir des maladies liées à un stress oxydant et/ou une inflammation et la composition de l'invention à une utilisation non thérapeutique pour renforcer l'action des autres antioxydants utilisés chez un animal. La composition de l'invention est également destinée à à une utilisation non thérapeutique pour stimuler la vitalité des cellules chez un animal.

Dans un mode de réalisation, la composition selon l'invention pour son utilisation en tant que médicament est administrée en association avec un autre médicament. De préférence, ce médicament est aussi est destiné à traiter ou prévenir des maladies liées à un stress oxydant et/ou une inflammation et/ou à renforcer l'action d'autres molécules pharmaceutiques, et en particulier d'autres antioxydants utilisés chez un animal. Selon un mode particulier, ce médicament est également destiné à traiter la mucoviscidose ou l'ataxie de Friedrich.

Dans un mode de réalisation de l'invention, l'animal est choisi parmi le groupe des animaux de rente, de compagnie ou de laboratoires, avantageusement choisi parmi le groupe constitué par les porcins, les bovins, les chevaux, les ovins, les caprins, les cervidés, les volailles, les lapins, les animaux aquacoles, les poissons, les crustacés, avantageusement les crevettes et les crabes, les reptiles, avantageusement les tortues et crocodiles, les oiseaux, les félins, les rongeurs, avantageusement les souris, les rats et les cobayes, et les canidés, de façon avantageuse parmi les ruminants et les monogastriques. Chez de tels animaux, la maladie liée à un stress oxydant et/ou une inflammation est de préférence choisie parmi les inflammations chroniques telles que l'arthrose, les tendinites, la fourbure, les maladies auto-immunes, les mammites entraînant la présence de cellules dans le lait, la mortalité embryonnaire lors de la reproduction des animaux, les effets du stress provoqués par un changement de température, d'alimentation, de densité d'élevage, d'habitat, lors de compétitions, d'exercice intense et/ou prolongé, comme par exemple le travail, la marche ou la course, de vaccinations, d'alimentation intensive ou lors des périodes de transition telles que la mise à bas, la ponte, le sevrage, le changement de bassin, le démarrage en élevage, par exemple la mise en nurserie, poussinière ou alevinage, l'allaitement, le transport, l'allotement, les troubles de la reproduction des animaux, par exemple une mauvaise maturation des follicules et/ou des ovules, les troubles immunitaires spécifiques et non spécifiques des animaux, notamment les agressions microbiennes, virales, parasitaires, et/ou le manque de résistance de leur organisme aux maladies, et les surcharges oxydantes.

Dans un autre mode de réalisation de l'invention, l'animal est l'homme. Dans ce cas, les maladies liées à un stress oxydant et/ou une inflammation sont avantageusement choisies parmi le groupe constitué des allergies, par exemple l'eczéma, le vitiligo, le lupus, les troubles liés à une exposition à une radiation UV, les dysfonctionnements post irradiation, en particulier les fibroses d'origine variée et la cystite, la stérilité, les pathologies respiratoires, en particulier l'asthme, l'anémie, les pathologies arthritiques rhumatoïdes et ostéoarthrites, en particulier l'ostéoarthrite du genou ou du doigt, les désordres génito-urinaires, en particulier la maladie de Peyronie, les problèmes liés à l'ischémie-reperfusion, les pathologies du système nerveux, en particulier l'hypoxie, les maladies cardiovasculaires, en particulier l'hypertension, l'obésité, le diabète de type II, le traitement du cancer, notamment les cancers colorectaux ou les mélanomes, les inflammations colorectales, notamment la maladie de Crohn, les pathologies neurodégénératives, notamment la maladie d'Alzheimer, Parkinson ou la sclérose latérale amyotrophique, les colites ulcératives, les troubles de la vision, par exemple la DMLA, les dysfonctionnements mitochondriaux, les dégénérescences dues à un agent infectieux, notamment le SIDA et l'hépatite C, ou les dégénérescences liées à l'utilisation d'un médicament ou à l'exposition à un produit chimique toxique, comme par exemple les pesticides. On peut également citer les maladies orphelines, la mucoviscidose, l'ataxie de Friedrich et l'alopécie. On peut en outre citer l'amélioration de la fertilité, l'amélioration de la vitalité des cellules souches permettant une meilleure utilisation et efficacité dans les thérapies cellulaires, en particulier pour les maladies inflammatoires articulaires, maladies de la moelle osseuse, et les maladies neurodégénératives telles que la maladie d'Alzheimer.

Enfin, le mélange selon la présente invention, du fait de son action anti-oxydante particulièrement avantageuse, est également utile dans le traitement des myopathies (pour une reference faisant le lien entre myopathie et stress oxidant, voir Functional muscle impairement in fascioscapulohumeral muscular distrophy is correlated with oxidative stress and mitochondrial dysfunction. Turki et al, 2012. Free Radical Biology and Medicine : 1 :53(5) 1068-1079).

En outre, comme indiqué plus haut, le mélange de SOD selon l'invention module de manière bénéfique l'expression des gènes NPPA et RXFP1 chez l'animal, en particulier chez l'homme.

Le gène NPPA code pour une protéine qui appartient à la famille des peptides natriurétiques, impliqués dans le volume des fluides extracellulaires et de l'homéostasie des électrolytes. Le gène humain NPPA code pour une préprohormone de 151 acides aminés (AA). Après clivage, de la séquence signal N-term de 25AA, la prohormone qui en résulte (pro ANP) est de 126 AA et est stockée dans des granules de sécrétion auriculaire. Lorsqu'elle est sécrétée la proANP est transformée en ANP (Atrial Natriuretic Peptide) active (1).

La sécrétion d'ANP dépend essentiellement d'un stimulus mécanique ; Ce sont les variations de la volémie qui régulent l'ANP par l'intermédiaire des variations de tension pariétale auriculaire, et favorise ainsi par son action, la baisse de la pression artérielle. L'ANP a donc une action anti-hypertrophique. En cas d'hypertrophie ou d'insuffisance cardiaque, la pro ANP et l'ANP sont sécrétés dans le myocarde du ventricule, entrainant leur augmentation dans le plasma en cas de maladie cardiaque (1).

L'ANP a un rôle dans la régulation de l'électrophysiologie cardiaque en agissant sur le système nerveux autonome, en inhibant l'activité sympathique, en activant l'activité parasympathique et en faisant la régulation spécifique des canaux ioniques cardiaques (2). Le gène NPPA constitue une cible thérapeutique pour réguler sélectivement la progression des maladies cardiovasculaires (1), car il est stimulé en cas de pathologie cardiaque (3). Les maladies associées au gène NPPA sont l'infarctus aigu du myocarde, les maladies de la valve mitrale, l'insuffisance cardiaque, l'hypertension, la surcharge fluidique ou encore l'hypertrophie cardiaque. Des mutations de ce gène sont associées avec une fibrillation auriculaire (2). Une expression de NPPA supérieure dans le coeur est impliquée dans la pathophysiologie de l'hypertension et de l'insuffisance cardiaque. (3)

Le récepteur RXFP1, aussi connu sous le nom de LGR7 (Leucine-rich-repeat containing G-protein-coupled Receptor 7), est un polypeptide de 757 acides aminés faisant partie de la superfamille de l'insuline. C'est un récepteur transmembranaire qui lie la relaxine avec une haute affinité. Il est couplé à une protéine G et possède 7 domaines transmembranaires. De plus, son ectodomaine possède 10 « répétitions riches en leucine » (« Leucine Rich Repeats », LRR) et est constitué de lipoprotéines de classe A : LDLa (Low Density Lipoprotein a) en amine terminal, ce qui influence la maturation des récepteurs, leur positionnement à la surface cellulaire et la signalisation de la relaxine [L]. Ce récepteur est donc à la base de la signalisation cellulaire et de la transduction du signal. Les récepteurs à la relaxine se trouvent dans les tissus reproductifs, le cerveau, les reins, le coeur, les poumons où l'action de la relaxine a été établie (4). Au niveau cardiaque, les récepteurs RXFP1 se trouvent en premier lieu au niveau de l'oreillette, plutôt que dans le ventricule. Toutefois, il est établi que l'expression des ARNm de RXFP1 a également lieu dans le ventricule des rats, souris et de l'humain. La haute densité de RXFP1 au niveau de l'oreillette est en lien avec la réponse inotrope et chronotrope de la relaxine.

Suite à l'activation des récepteurs RXFP1, et à la transduction du signal, la relaxine va avoir différents effets. La fonction de la relaxine n'est pas confinée au système reproductif, ce peptide affecte la fonction cardiaque et participe entre autre, à la régulation de la pression sanguine, l'écoulement sanguin, la balance fluidique (5). La relaxine a des effets antifibrotiques, antihypertrophiques, ant-inflammatoires et vasodilatateurs. Elle réduit également la pression artérielle et augmente le débit cardiaque, et elle peut également stimuler la régénération du myocarde (4) (5).

Les taux de mRNA RXFP1 sont diminués dans l'oreillette et le ventricule gauche chez le rat MI (myocard Infact) (4).

Ainsi, le mélange de SOD selon la présente invention est avantageusement utilisé pour le traitement des maladies cardiovasculaires, en particulier l'infarctus aigu du myocarde, les maladies de la valve mitrale, l'insuffisance cardiaque, l'hypertension, la surcharge fluidique, l'hypertrophie cardiaque, l'hypertension et de l'insuffisance cardiaque, en particulier chez l'homme.

Le mélange de SOD selon la présente invention est également avantageusement utilisé pour le traitement de l'obésité et de l'artériosclérose.

En outre, il a été constaté que le mélange de SOD selon la présente invention est utile pour le traitement de l'herpès, notamment l'herpès labial, en particulier chez l'homme.

La présente invention concerne également une composition selon l'invention pour son utilisation en tant que composition nutritionnelle, alimentaire ou cosmétique. Dans ce cas, la composition vise à lutter contre les effets du stress oxydant au quotidien tel que par exemple : la fatigue, le stress, l'anxiété, les problèmes articulaires, la récupération sportive, les problèmes de fertilité masculines ou féminine, l'érection, les problèmes de vues, la cicatrisation, la cellulite, les UV, l'acné.

### FIGURES

Figure 1 : Détermination des différentes SOD issues du melon hybride F1 MA 7950 (représentation schématique du résultat obtenu après révélation sur gel d'acrylamide).
Activité SOD totale (puits 1), avec KCN 2mM (puits 2), avec H₂O₂ (puits 3).
Figure 2 : profil électrophorétique des différentes SOD issues du melon hybride F1 MA 7950 (représentation schématique du résultat obtenu après révélation sur gel d'IEF), de gauche à droite, du melon MA 7950 (puits 1 et 2), de pastèque (puits 3 et 4), de pêche (puits 5 et 6) et de nectarine (puits 7 et 8).
Figure 3 : profil électrophorétique des différentes SOD issues du melon hybride F1 MA 7950 (représentation schématique du résultat obtenu après révélation sur gel d'IEF), de gauche à droite, du melon MA 7950 (puits 1 et 2), de melon Canari (puits 3 et 4), d'abricot (puits 5 et 6) et de cerise (puits 7 et 8).
Figure 4 : Photographie de deux melons, juste après la cueillette (t0). A gauche se trouve un melon hybride F1 de type commun, à droite se trouve un melon issu de la variété de melon hybride F1 nommée MA 7950.
Figure 5 : Photographie des deux mêmes melons prise 7 jours plus tard. A gauche se trouve le melon hybride F1 de type commun, à droite se trouve le melon issu de la variété de melon hybride F1 nommée MA 7950.

### EXEMPLES

Les exemples qui suivent sont présentés à titre illustratifs et ne constituent en aucun cas une limitation de l'invention.

### Exemple 1. Spécificité de la variété de melon hybride F1 nommée MA 7950

Une expérience simple a été mise en œuvre afin de démontrer les propriétés particulière des melons issus de la variété de melon hybride F1 nommée MA 7950, et en particulier de son activité antioxydante.

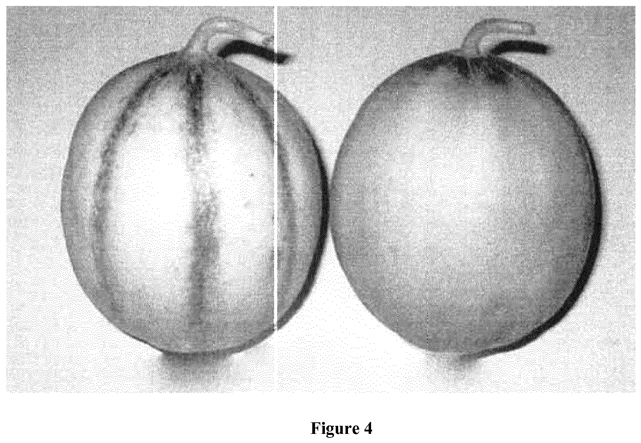

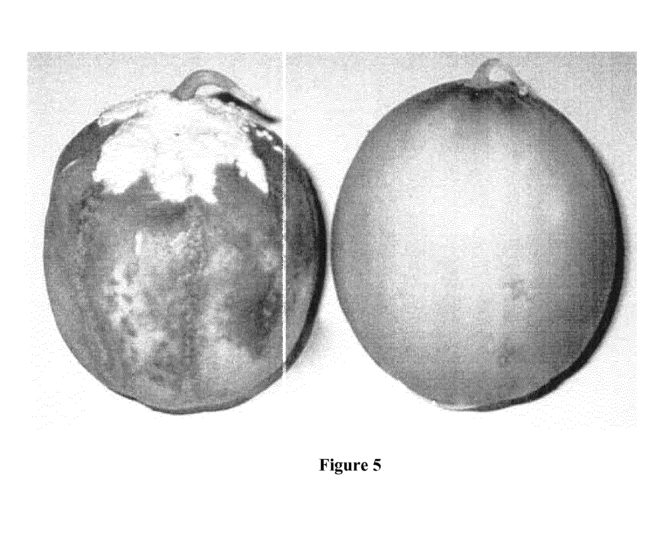

Deux melons, l'un de type commun, l'autre issu de la variété de melon hybride F1 nommée MA 7950, ont été déposés dans une pièce à température ambiante et pression atmosphérique au jour J1 (figure 4). Les melons sont conservés dans cette pièce dans les mêmes conditions (température ambiante, pression atmosphérique) pendant 7 jours (figure 5, photographie des mêmes melons prise au jour J8). Après 7 jours, on voit bien que le melon hybride F1 de type commun est en état avancé de dégradation, tandis que le melon issu de la variété de melon hybride F1 nommée MA 7950 présente toujours un bon aspect extérieur. La teneur deux fois plus importante et la composition du mélange de SOD de la variété de melon hybride F1 nommée MA 7950 permet aux cellules du melon de résister plus efficacement au processus de dégradation naturelle.

### Exemple 2. Préparation de l'extrait et purification des différentes SOD, dosage de l'activité SOD

5g de pulpe d'un Cucumis melo de variété hybride F1 nommée MA 7950 ou de ses cellules sont broyés dans un mortier à froid. Un volume tampon Phosphate 50 mM (pH :7,8 ; EDTA 1 mM ; glycérol 5 %) équivalent à 3 fois la masse végétal est ajouté. Après homogénéisation, la suspension est centrifugée à 5000 g à 4°C pendant 30 minutes. Le surnageant est ensuite récupéré et filtré. Cet extrait brut sert à la purification des différentes formes de SOD. Les différentes SOD ont été purifiées selon une technique bien connue de l'homme de l'art par IMAC (immobilised metal affinity chromatography). Dans cette méthode, le cuivre est utilisé comme ion métal immobilisé sur la colonne, l'accrochage des protéines est dépendante de leur charge, et est donc corrélée à la présence d'acides aminés comme le tryptophane, l'histidine, la cystéine. Les SOD sont connues pour leur richesse en histidine, de ce fait l'utilisation de IMAC est justifiée.

Les SOD provenant de l'extrait de melon MA 7950 ont été purifiées sur une FPLC (Pharmacia Amersham) en utilisant une colonne Superose HR 10/2 ou Hitrap chelating colonne (pharmacia-Biotech). 3 ml d'échantillon sont injectés par une superloop sur la colonne préalablement traitée par une solution de CuSO4 (600ml, 23mmol/ml) et équilibrée avec 10 ml de tampon phosphate de potassium 0.05 M pH 7.8. L'élution des SOD est effectuée à débit constant de 1ml/ min par un gradient linéaire de 10% (atteint en 10 min) d'une solution de NH₄Cl 0,75 M. Le reste des complexes Cu-protéines est éliminé par 5 ml d'une solution aqueuse d'EDTA 1 M. Des fractions de 2 ml contenant les différentes SOD sont recueillies, dosées et immédiatement déssalées sur cellule Amicon PM 10 avec 4 volumes de tampon phosphate 0.05 M pH 7,8, puis sont lyophilisées.

### Exemple 3. Détermination des différentes formes de SOD et poids moléculaires:

L'activité SOD est révélée sur gel d'acrylamide en conditions natives (conditions dans lesquelles la protéine reste à l'état natif, c'est-à-dire telle quelle et dans la cellule, par opposition à des conditions dénaturantes dans lesquelles la protéine est linéarisée) selon la méthode de Beauchamp et Fridovich Anal. Biochem. 44 :276-82 (1971). Elle est basée sur l'inhibition de la réduction du Nitroblue Tetrazolium (N 55 14 Sigma-Aldrich, France) par la SOD. Après révélation, les différentes bandes correspondant à l'activité SOD apparaissent en blanc sur un fond de gel bleu foncé. Des échantillons du mélange de SOD purifiées (45 µl de solution A + 15 µl de tampon de migration) sont déposés dans les différents puits d'un gel d'électrophorèse (4% / 10% d'acrylamide). La migration s'effectue pendant 75 min à 70mA et 300V. Lors de l'étape de révélation du gel qui a migré en conditions non dénaturantes, le gel est découpé en 3 parties. La première est révélée en activité selon le protocole ci-dessous, la deuxième est préincubée avec 2 mM de KCN puis révélée en activité, enfin la dernière est préincubée avec 5 mM de H₂O₂ puis révélée en activité.

### Conditions de révélation de l'activité SOD:

L'activité des SOD purifiées est mesurée à l'aide du kit d'analyse SOD assay kit (19160, Sigma-Aldrich, France). Cette activité SOD totale est supérieure à 500 UI SOD/mg protéines et la teneur en protéines de l'extrait purifié est supérieure à 70% (méthode Bradford, Anal. Biochem. 72 : 248-54, 1976). Après migration, les gels sont plongés :
- pendant 150 min dans un tampon phosphate de K⁺ (50 mM ; pH 7,8) contenant 2 mM NBT.
- puis pendant 15 min dans un tampon phosphate de K⁺ (50 mM ; pH 7,8) contenant 28 mM TEMED et 0,0028 mM riboflavine (préparer une solution de riboflavine 50 mg dans 1ml de tampon phosphate).

Les gels sont ensuite rincés dans une solution de tampon phosphate de K (50 mM ; pH 7,8). Les gels d'électrophorèse sont ensuite numérisés à l'aide du module de capture photographique Perfect Image, puis analysés par le module d'analyse Gel analyst (Claravision, 2000, France).

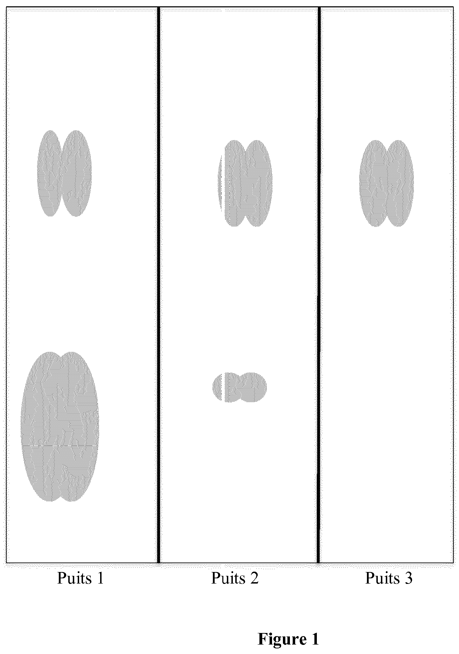

Comme on le voit sur la figure 1, en l'absence d'inhibiteurs, le mélange de SOD présente deux épaisses bandes d'activité SOD (puit 1), une située vers le haut du gel, indiquant des protéines de poids moléculaire élevé (qui migrent moins vite que les protéines de faible poids moléculaire) et une vers le bas du gel indiquant une activité due à une ou des SOD de faible poids moléculaire.

En présence de 2 mM KCN (puit 2), seule la Cu/Zn-SOD est inhibée, cela correspond à la bande située au bas du gel et cela représente la plus grande partie de cette bande d'activité.

En présence de 5 mM H₂O₂ (puit 3), la Fe-SOD et la Cu/Zn-SOD sont inhibées. La seule bande qui reste sur le gel (en haut) correspond à la Mn-SOD (insensible à KCN et H₂O₂). La Mn-SOD represente une part de la bande d'activité SOD située en haut du gel, l'autre partie est une Fe-SOD inhibée par H₂O₂. L'autre forme de Fe-SOD est située en bas du gel, près de la Cu/Zn-SOD.

Les intensités des différentes bandes d'activité SOD sont intégrées à l'aide du module d'analyse Gel Analyst (Claravision, 2000, France), le ratio de chaque isoforme de SOD est ainsi calculé :

| Intensité de chaque bande | SOD sans inhibiteurs | +KCN | +H₂O₂ |
|---|---|---|---|
| 1 | 177 160 | 173 721 | 62 124 |
| 2 | 442 524 | 43 594 | / |
| Total | 619 684 | 217 315 | 62 124 |

| Résultats en % | SOD sans inhibiteurs | +KCN | +H₂O₂ |
|---|---|---|---|
| 1 | 28,6 | 28, | 10 |
| 2 | 71,4 | 7 | / |
| Total | 100 | 35 | 10 |

Pour la Mn-SOD (bande 3), l'intensité de la bande est 62 124 pour une intensité totale de 619 684 (SOD sans inhibiteurs), la Mn-SOD représente donc 10% de l'activité SOD totale. Pour la Cu/Zn-SOD, l'intensité de la bande est (442 524-43 594)/619 684 et correspond à 65% de l'activité totale. Enfin, la Fe-SOD correspond à 25%, une isoforme représente 7% (près de la Mn-SOD) et l'autre 18% (près de la Cu/Zn-SOD).

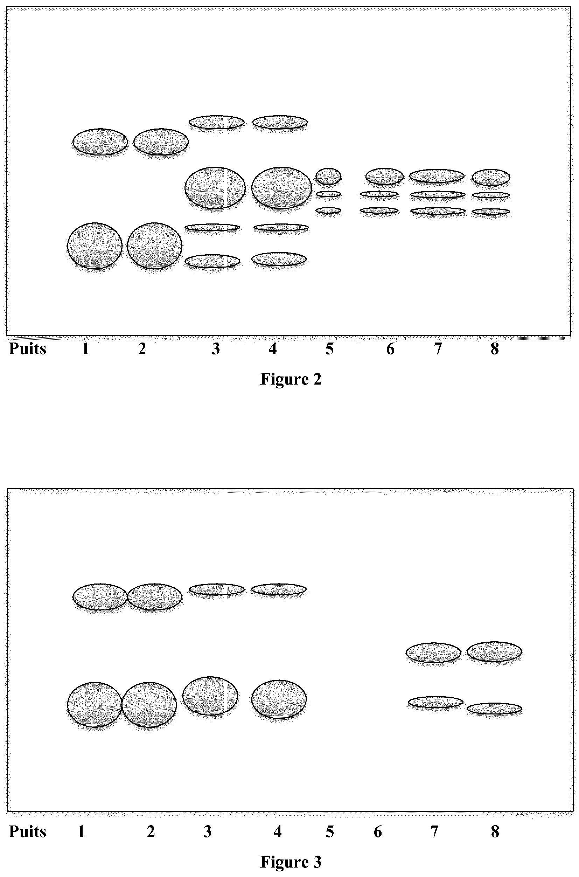

Ces analyses ci-dessus faites sur un mélange de SOD extrait de la variété hybride F1 de Cucumis Melo MA 7950, ont été réalisées dans les mêmes conditions sur d'autres SOD provenant de différents fruits : melon Anasta, melon espagnol (Canari), melon Clipper (descendant de la variété hybride F1 de Cucumis Melo 95LS444), pastèque, pêche, nectarine, cerise (Figures 2 et 3).

Comme on le voit sur les figures 2 et 3, les profils d'électrophorèse des activités SOD provenant de différents fruits sont très différents un mélange de SOD purifiées extrait de la variété hybride F1 de Cucumis Melo MA 7950 (le plus à gauche).

Les pourcentages de chaque forme après utilisation d'inhibiteurs (2 mM KCN et 5 mM H₂O₂) sont reportés sur le tableau 1.

**Tableau 1 : Proportion des différentes formes de SOD.**

| | Cu/Zn-SOD | Mn-SOD | Fe-SOD |
|---|---|---|---|
| **Melon MA 7950** | **65%** | **10%** | **25%** |
| Pastèque | 66% | 18% | 16% |
| Melon Canari | 83% | 9% | 8% |
| Melon Anasta | 84% | 13.5% | 2.5 % |
| Melon Clipper | 25% | 60% | 15 % |
| Nectarine | 81.5% | 0% | 18.5% |
| Pêche | 88% | 0% | 12% |
| Cerise | 100% | 0% | 0% |
| Abricot | 0% | 0% | 0% |

Sur ces mêmes gels d'électrophorèse, des marqueurs de poids moléculaires (protéines de poids moléculaires connus), ont été introduits dans le premier puits du gel, en utilisant un kit de marqueurs (M 3913, Sigma-Aldrich, France) afin d'étalonner le gel en poids moléculaire et de déterminer par comparaison les poids moléculaires des différentes SOD.

Les mêmes analyses ont été réalisées sur des mélanges de SOD purifiées à partir de différents fruits, seulement sur les fruits contenant 3 formes de SOD : pastèque, melon espagnol (Canari), autre variété de melon (Anasta).

Les poids moléculaires des différentes SOD sont reportés sur le tableau 2.

**Tableau 2 : Poids moléculaires des différentes formes de SOD extrait de la variété hybride F1 de Cucumis Melo MA 7950, de la pastèque, du melon Canari, du melon Anasta et du melon Clipper (descendant de la variété hybride F1 de Cucumis Melo 95LS444).**

| | Cu/Zn SOD | Mn SOD | Fe SOD |
|---|---|---|---|
| **Melon MA 7950** | **31 800** | **80 600** | **32 200** |
| | | | **79 800** |
| Pastèque | 41 600 | 72 200 | 56 400 |
| Melon Canari | 44 700 | 65 900 | 43 700 |
| Melon Anasta | 42 600 | 62 700 | 44 700 |
| Melon Clipper | 40 000 | 95 000 | 30 000 |

On peut aisément constater que le un mélange de SOD purifiées extrait de la variété hybride F1 de Cucumis Melo MB17415 est unique (par comparaison aux autres SOD issues de différents fruits), notamment du fait de la présence de la seconde isoforme de la Fe-SOD. Il contient 3 classes de SOD (différenciées par leur groupement métal) et 4 isoformes :
- Cu/Zn-SOD représente 65% de l'activité SOD totale et a un poids moléculaire de 31 800 Da.
- Mn-SOD représente 10% de l'activité SOD totale et a un poids moléculaire de 80 600 Da.
- 2 différentes formes de Fe-SOD représentent 25% de l'activité totale :
   Une Fe-SOD de 32 200 Da représente 18% de l'activité SOD totale. L'autre Fe-SOD de 79 800 Da représente 7 % de la SOD totale.

### Détermination des points isoélectriques des différentes SOD

Cette technique d'analyse est intéressante pour l'obtention d'informations complémentaires sur le pHi des isoformes des SOD purifiées à partir du melon. Elle sera donc effectuée en activité. La séparation des SOD en fonction de leur charge se fait par isoélectrofocalisation (IEF). Les SOD migrent dans un champ électrique et s'immobilisent quand l'environnement de pH rend leur charge globale nulle. Les points isoélectriques des différentes formes de SOD peuvent être déterminés sur les gels d'IEF par comparaison à des marqueurs de point isoélectrique connu (IEF markers 3.6-9.3, Référence 56733, Sigma-Aldrich, France)

Les échantillons déposés sur gels (acrylamide/acrylamide bis 30%, glycérol 50%, ampholines 3.5-10 Amersham, persulfate d'ammonium et Temed) migrent à 300 V et 20 mA dans un tampon d'échantillon (glycérol 75% v/v, ampholines 2% v/v) et un tampon de migration (NaOH 25 mM pour la cathode et CH3COOH 25 mM pour l'anode). Des marqueurs de point isoélectrique sont également déposés sur un puits du gel. La révélation des bandes d'activité SOD se fait comme décrit dans le paragraphe 2. Par comparaison avec les bandes de marqueurs de pHi, les pHi des différentes isoformes sont déterminés. Pour le melon Anasta, la faible proportion de Fe-SOD (2.5%) ne permet pas l'identification des différents pHi. Les points isoélectriques des différentes formes de SOD des 3 variétés (MA 7950 ; pastèque, melon Canari) sont reportés sur le tableau 3.

**Tableau 3 : points isoélectriques (pHi) des différentes formes de SOD extrait de la variété hybride F1 de Cucumis Melo MA 7950, de la pastèque, du melon Canari.**

| | Cu/Zn SOD | Mn SOD | Fe SOD |
|---|---|---|---|
| **Melon MA 7950** | **4.3** | **4.1** | **4.4** |
| | | **4.4** | **4.7** |
| | | **5.3** | |
| | | **5.5** | |
| | | **5.7** | |
| | | **5.85** | |
| | | **6.1** | |
| Pastèque | 4.6 | 4.25 | 4 |
| | | 4.7 | 4.7 |
| | | 5.2 | |
| | | 5.8 | |
| | | 6 | |
| | | 6.1 | |
| Melon Canari | 4.4 | 4.4 | 4 |
| | | 5.7 | |
| | | 5.8 | |
| | | 5.9 | |
| | | 6 | |

On remarque que le mélange de SOD purifiées extrait de la variété hybride F1 de Cucumis Melo MA 7950 caractérisées par leur groupement métal, leur poids moléculaire et leur pHi est unique, il contient :
- Cu/Zn-SOD représente 65% de l'activité SOD totale, a un poids moléculaire de 31 800 Da et un pHi de 4.3 ;
- Mn-SOD représente 10% de l'activité SOD totale, a un poids moléculaire de 80 600 Da et a 7 isoformes de pHi différents compris entre 4 et 6 ;
- 2 différentes formes de Fe-SOD représentent 25% de l'activité totale :
   Une Fe-SOD de 32 200 Da représente 18% de l'activité SOD totale. L'autre Fe-SOD de 79 800 Da représente 7% de l'activité SOD totale. Elles ont un pHi de 4.4 et 4.7.

Dans 1 mg du mélange selon l'invention, il y a environ 0.71 mg de Cu/Zn SOD, 0.06 mg de Mn-SOD et 0.23mg de Fe-SOD.

### Exemple 4. Efficacité du mélange de SOD purifiées selon l'invention sur un marqueur de stress oxydant : l'anion superoxyde (O₂°-) produit par la NADPH oxydase et sur un marqueurle TNP α

Dans les macrophages, l'utilisation de molécules comme l'interféron gamma ou le LPS permettent d'induire un burst oxydatif (surproduction du radical superoxyde dû à une stimulation de la NADPH-oxydase) et une réponse inflammatoire (augmentation de la production de TNF α et d'interleukines).

L'effet de différentes doses d'un mélange de SOD purifiées selon l'invention sur la production du radical superoxyde (O₂˙-), et sur la production d'une cytokine inflammatoire (le TNF α) sur une lignée cellulaire de macrophages murins RAW 264.7 a été évalué.

Les cellules sont cultivées dans un milieu RPMI (Roswell Park Memorial Institute Medium) additionné de sérum de veau foetal (10%, Life Technologies), d'une solution d'antiobiotiques penicilline-streptomycine (1%, Life Technologies) et de fungizone (0,25%, Life Technologies) dans des boîtes de culture T 175 cm² (Dutscher, Brumath, France) en atmosphère humide enrichie à 5% de CO₂ à 37°C. Les cellules sont comptées et repiquées tous les trois jours à l'aide d'une lame de Thoma puis sont réparties dans des boîtes de culture à la concentration de 250 000 cellules/ ml. Les cellules sont ensuite incubées avec un mélange de SOD purifiées selon l'invention (de 0 à 100 UI SOD/ml) pendant 12 heures. Elles sont ensuite décrochées du support et suspendues à une concentration de 10⁶ cellules/ml dans le RPMI. On place 890 µl de cette suspension en présence de 100 µl de lucigénine 1 mM et on incube à 37°C pendant 30 minutes. A l'issu des 30 minutes et au moment de la mesure, on ajoute 10 µl de PMA sont ajouté (Phorbol-12-Myristyl-13-Acétate, Sigma Chemical; St louis Mo; USA) 10⁻⁷ M pour la mesure de la prduction du radical superoxyde. Pour stimuler la production de TNF-α, du lipopolysaccharide (LPS) est introduit 12h avant la mesure dans les puits.

La production d'O₂°- est mesurée par une technique de chimioluminescence (Chen et al., Am. J. Renal. Physiol. 289 F 749-53 2005) en présence de lucigénine (10 µM), sonde de bioluminescence spécifique de l'anion superoxyde. Brièvement, en présence d'O₂°⁻, la lucigénine va passer d'un état fondamental à un état excité. Lors de son retour à l'état fondamental, la lucigénine va émettre des photons. La quantité de photons émise sera proportionnelle à la formation d'O₂°⁻. L'intensité de la luminescence est enregistrée par un lecteur microplaques à luminescence (Synergy 2 Biotek, USA). Les résultats sont exprimés en coups/mg de protéines.

La production de TNF α dans les macrophages est mesurée par ELISA à l'aide d'un kit d'analyse DY 510 (R&D system, Mineapolis, USA). Les résultats sont la moyenne de 3 répétitions indépendantes.

Comme on le voit sur les tableaux 4 et 5, le mélange de SOD issues de la lignée MA 7950 modulent la réponse oxydative et inflammatoire de la lignée de macrophages murins en inhibant la production de l'anion superoxyde par la NADPH oxydase et la production de TNFα.

**Tableau 4 : Effet de différentes doses un mélange de SOD purifiées extrait de la variété hybride F1 de Cucumis Melo MA 7950 sur la production de l'anion superoxyde**

| | | | | | |
|---|---|---|---|---|---|
| (SOD (UI/ml) | 0 | 5 | 10 | 50 | 100 |
| % d'activation | 100± 5 | 79.5± 5.5 | 66.4± 3.2 | 30.5± 2.5 | 19.6± 2.4 |

**Tableau 5 : Effet de différentes doses du mélange de SOD de MA 7950 sur la production du TNF α**

| | | | | | |
|---|---|---|---|---|---|
| (SOD (UI/ml) | 0 | 5 | 10 | 50 | 100 |
| TNF α (pg/ml) | 721± 12 | 454±17 | 166±16 | 69±7 | 59±5 |

### Exemple 5 : efficacité du mélange de SOD purifiées selon l'invention sur la synthèse endogène d'enzymes antioxydantes

Un mélange de SOD purifiées selon l'invention a été encapsulé selon le procédé décrit dans la demande internationale WO2006030111 pour permettre une utilisation par voie orale.

10 rats mâles Wistar (élevage Janvier, Le Genest-St-Isle, France) âgés de 3 semaines ont été utilisés. Dès leur arrivée, ils sont répartis au hasard en groupes, puis sont hébergés dans des cages en plastique dans un environnement contrôlé avec une température de 23±1°C, une hygrométrie de 70% et une photopériode de 12 heures (12h d'éclairement/ 12h d'obscurité). Les rats ont été manipulés selon les directives NIH (National Research Council).

Les animaux ont un accès libre à la nourriture et à l'eau. La consommation alimentaire est mesurée quotidiennement et le poids corporel des animaux est enregistré tous les deux jours.

Les animaux sont séparés en 2 groupes de 5.

Un groupe reçoit le régime standard (EF R/M control E 15000-00, SSNIFF, Germany). L'autre groupe reçoit le régime standard (EF R/M control E 15000-00, SSNIFF, Germany) ainsi qu'une dose de 4 UI SOD (ou U/mg SOD) du mélange selon l'invention par jour pendant 28 jours.

A la fin des 28 jours d'expérience, les animaux sont anesthésiés à l'aide d'une injection intrapéritonéale de pentobarbital. Le foie est perfusé avec 0.15M de NaCl, puis prélevé et stocké à -80°C.

L'expression des enzymes antioxydantes SOD et GPx a été déterminée par western-blot.

Les résultats sont reportés sur le tableau 4. Comme on le voit, l'ingestion du mélange de SOD issues du melon MA 7950 entraîne l'induction de l'expression des enzymes antioxydantes SOD et GPx chez le rat Wistar et ce en absence de stress oxydant.

**Tableau 6 : Quantification de l'expression de la SOD et de la glutathion peroxydase (GPX) dans le foie de rats mâles Wistar**

| | **SOD** | **GPX** |
|---|---|---|
| animaux témoin | 100 | 100 |
| animaux traités avec un mélange de SOD purifiées selon l'invention | 235± 8 | 161±5 |

En situation de stress oxydant, ce stock de protéines antioxydantes peut ensuite être activé et utilisé, ce qui permet une meilleure réponse des cellules au stress oxydant.

### Exemple 6 : effet du mélange de SOD selon l'invention sur l'expression des gènes NPPA et RXFP1

L'effet du mélange de SOD purifiées selon l'invention sur les gènes impliqués dans l'hypertrophie cardiaque a été évalué sur un modèle de rats spontanément hypertendus (SHR). Il s'agit de rats spontanément hypertendus et présentant une hypertrophie cardiaque, issus de croisements génétiques, qui sont utilisables dès leur réception et qui présentent l'avantage d'être « prêts à l'emploi ».

Les différents mélanges de SOD purifiées selon l'invention ont été encapsulés selon le procédé décrit dans la demande internationale WO2006/030111 pour permettre une utilisation par voie orale.

40 rats mâles SHR (élevage Janvier, Le Genest-St-Isle, France) âgés de 3 semaines ont été utilisés. Dès leur arrivée, ils sont répartis au hasard en groupes, puis sont hébergés dans des cages en plastique dans un environnement contrôlé avec une température de 23±1°C, une hygrométrie de 70% et une photopériode de 12 heures (12h d'éclairement/ 12h d'obscurité). Les rats ont été manipulés selon les directives NIH (National Research Council).

Les animaux ont un accès libre à la nourriture et à l'eau. La consommation alimentaire est mesurée quotidiennement et le poids corporel des animaux est enregistré tous les deux jours.

Les animaux sont séparés en 4 groupes de 10 (groupes 1, 2, 3 et 4).

Le groupe 1 reçoit le régime standard (EF R/M control E 15000-00, SSNIFF, Germany). Le groupe 2 reçoit pendant 4 jours, le régime standard (EF R/M control E 15000-00, SSNIFF, Germany) ainsi qu'une dose de 4 UI SOD (ou U/mg SOD) par jour du mélange de SODs issues du melon MA7950 selon l'invention.

Les groupes 3 et 4 reçoivent pendant 4 jours, le régime standard (EF R/M control E 15000-00, SSNIFF, Germany) ainsi qu'une dose de 4 UI SOD (ou U/mg SOD) par jour du mélange de SODs issues du melon Canari (groupe 3) ou Anasta (groupe 4)

A la fin des 4 jours d'expérience, les animaux sont anesthésiés à l'aide d'une injection intrapéritonéale de pentobarbital. Le cœur est prélevé et l'ANP et RXFP1 sont dosés par western blot sur le ventricule gauche (VG).

L'extraction protéique du VG a été réalisée dans la glace avec du tampon Tris 20mM (pH 6.8) contenant 150mM NaCl, 1mM EDTA, 1% Triton 20%, 0.1% SDS, et 1% de cocktail d'inhibiteurs de protéases. Après centrifugation (1500 rpm, 15 min à 4°C), le surnageant a été collecté et les protéines des tissus ont été séparées par SDS PAGE. Une quantité équivalente de protéines a été déposée sur des gels à 10 ou 15% d'acrylamide avec un gel de concentration à 4% d'acrylamide. La migration a été faite dans du tampon Tris-glycine-SDS provenant de Sigma-Aldrich (Saint Quentin Fallavier, France). Après séparation, les protéines ont été transférées sur des membranes de nitrocellulose (Whatman, Dassel, Allemagne).

La quantification a été faite après standardisation dans les membranes en exprimant la densité de chaque bande d'intérêt relative à la GAPDH dans une même ligne. Les résultats sont ensuite exprimés en tant que changements relatifs par rapport au groupe placebo. ** p<0.01 comparé au groupe placebo.

Les résultats sont reportés sur le tableau 7 ci-dessous.

**Tableau 7: Quantification de l'expression de l'ANP et de RXFP1 dans le cœur de rats SHR.**

| | ANP | RXFP 1 |
|---|---|---|
| Animaux témoins | 100 | 100 |
| Animaux traités avec mélange SOD issues du melon MA7950 | 54 ** | 147 ** |
| Animaux traités avec mélange de SOD issues du melon Canari | 98 (NS) | 103 (NS) |
| Animaux traités avec mélange de SOD issues du melon Anasta | 97 (NS) | 96 (NS) |
| (NS) : non significatif ; ** : significatif | | |

Comme on le voit sur le tableau 7 ci-dessus, seule l'ingestion du mélange de SOD issues du melon MA 7950 (mélange de SOD selon l'invention) entraîne une modulation de l'expression des protéines ANP et de RXFP1, dont les gènes NPPA et RXFP1 sont impliqués dans les pathologies cardiovasculaires, en particulier l'hypertrophie cardiaque, et l'obésité. Cette modulation, comme expliqué plus haut, est bénéfique dans le cas de maladies cardiovasculaires (diminution de la concentration en ANP et augmentation de la concentration en RXFP1).

## Revendications

1. Mélange de superoxyde dismutases d'origine végétale, **caractérisé en ce qu'**il est constitué essentiellement de 3 superoxyde dismutases : une superoxyde dismutase au manganèse, une superoxyde dismutase au cuivre et au zinc et une superoxyde dismutase au fer présente sous au moins deux isoformes, la première isoforme de superoxyde dismutase au fer avec un poids moléculaire compris entre 28 000 et 36 000 Da, la deuxième isoforme de superoxyde dismutase au fer avec un poids moléculaire compris entre 75 000 et 85 000 Da, ledit mélange étant susceptible d'être obtenu à partir d'un extrait de la variété hybride F1 de Cucumis Melo MA 7950 (numéro NCIMB 42154) ou de ses cellules cultivées in vitro, et **caractérisé en ce que** l'activité SOD cumulée des deux isoformes de superoxyde dismutases au fer est comprise entre 20% et 26% de l'activité SOD totale du mélange, l'activité de la superoxyde dismutase au cuivre et au zinc est comprise entre 60% et 70% de l'activité SOD totale du mélange, et l'activité de la superoxyde dismutase au manganèse est comprise entre 7 et 12% de l'activité SOD totale du mélange.

2. Mélange de superoxyde dismutases selon la revendication 1, **caractérisé en ce que** ledit mélange présente une activité SOD totale supérieure ou égale à 130 U/mg dudit mélange.

3. Mélange de superoxyde dismutases selon la revendication 1 ou 2, **caractérisé en ce que** la première isoforme de superoxyde dismutase au fer présente un poids moléculaire d'environ 32 200 Da.

4. Mélange de superoxyde dismutases selon l'une quelconque des revendications 1 à 3, **caractérisé en ce que** la deuxième isoforme de superoxyde dismutase au fer présente un poids moléculaire d'environ 79 800 Da.

5. Mélange de superoxyde dismutases selon la revendication 1, **caractérisé en ce que** l'activité SOD cumulée des deux isoformes de superoxyde dismutases au fer est comprise entre 22% et 26% de l'activité SOD totale du mélange.

6. Mélange de superoxyde dismutases selon l'une quelconque des revendications 1 à 5, **caractérisé en ce que** la superoxyde dismutase au manganese présente un poids moléculaire compris entre 70 000 et 90 000 Da et la superoxyde dismutase au cuivre et au zinc présente un poids moléculaire compris entre 27 000 et 33 000 Da.

7. Mélange de superoxyde dismutases selon l'une quelconque des revendications 1 à 6, **caractérisé en ce qu'**il est susceptible d'être obtenu par broyage ou pressage en milieu aqueux, de préférence à pH de 5 à 9, de la variété hybride F1 de Cucumis Melo MA 7950 ou de ses cellules cultivées in vitro puis récupération du surnageant et purification par chromatographie, en particulier par chromatographie IMAC.

8. Composition nutritionnelle, cosmétique ou pharmaceutique contenant à titre d'ingrédient actif un mélange de superoxyde dismutases selon l'une quelconque des revendications 1 à 6 et au moins un excipient alimentaire, pharmaceutiquement ou cosmétiquement acceptable.

9. Composition cosmétique selon la revendication 8 **caractérisée en ce qu'**elle est destinée à un usage topique externe telle que les produits de soins, les shampoings, les lotions, les gels.

10. Utilisation non thérapeutique d'une composition contenant à titre d'ingrédient actif un mélange de superoxyde dismutases selon l'une quelconque des revendications 1 à 6 et au moins un excipient acceptable, pour renforcer l'action d'autres antioxydants utilisés chez un animal.

11. Utilisation non thérapeutique d'une composition contenant à titre d'ingrédient actif un mélange de superoxyde dismutases selon l'une quelconque des revendications 1 à 6 et au moins un excipient acceptable, pour stimuler la vitalité des cellules chez un animal.

12. Composition pharmaceutique selon la revendication 8, **caractérisée en ce qu'**elle est destinée à une administration par voie topique, orale, nasale ou parentérale, et se présente par exemple sous forme de comprimé, de gélule, de capsule molle, de comprimé effervescent, de sachet ou de stick à diluer, de sirop, d'élixir, de tisanes, de gommes à mâcher, de spray, d'aérosol ou de solution injectable.

13. Composition pharmaceutique selon la revendication 8 ou 12, **caractérisée en ce qu'**elle contient un autre principe actif, avantageusement un autre antioxydant.

14. Composition pharmaceutique selon l'une quelconque des revendications 8, 12 ou 13 pour son utilisation en tant que médicament.

15. Composition pharmaceutique pour utilisation selon la revendication 14, **caractérisée en ce que** le médicament est destiné à traiter ou prévenir, chez un animal, des maladies liées à un stress oxydant et/ou une inflammation, ou à traiter la mucoviscidose ou l'ataxie de Friedrich.

16. Composition pharmaceutique pour utilisation selon la revendication 15, **caractérisée en ce que** l'animal est l'homme.

17. Composition pharmaceutique pour utilisation selon la revendication 14, **caractérisée en ce que** le médicament est destiné au traitement des maladies cardiovasculaires, de l'obésité, de l'artériosclérose, et de l'herpès labial, en particulier chez l'homme.

18. Composition nutritionnelle selon la revendication 8, **caractérisée en ce qu'**elle est destinée à une administration par voie orale et se présente par exemple sous forme de comprimé, de gélule, de capsule molle, de comprimé effervescent, de sachet ou de stick à diluer, de gommes à mâcher, de boissons, de jus, de yaourt, de confiserie, de biscuit ou de barres.

## Patentansprüche

1. Mischung aus Superoxiddismutasen pflanzlichen Ursprungs, **dadurch gekennzeichnet, dass** sie im Wesentlichen aus drei Superoxiddismutasen besteht: einer Mangan-Superoxiddismutase, einer Kupfer- und Zink-Superoxiddismutase und einer Eisen-Superoxiddismutase, die in mindestens zwei Isoformen vorliegt, die erste Isoform der Eisen-Superoxiddismutase mit einem Molekulargewicht zwischen 28.000 und 36.000 Da, die zweite Isoform der Eisen-Superoxiddismutase mit einem Molekulargewicht zwischen 75.000 und 85.000 Da, wobei die Mischung aus einem Extrakt der Hybridvarietät F1 von Cucumis Melo MA 7950 (Nummer NCIMB 42154) oder deren in vitro kultivierten Zellen gewinnbar ist, und **dadurch gekennzeichnet, dass** die kumulierte SOD-Aktivität der beiden Eisen-Superoxiddismutase-Isoformen zwischen 20% und 26% der gesamten SOD-Aktivität der Mischung liegt, die Aktivität der Kupfer- und Zink-Superoxiddismutase zwischen 60% und 70% der gesamten SOD-Aktivität der Mischung liegt, und die Aktivität der Mangan-Superoxiddismutase zwischen 7% und 12% der gesamten SOD-Aktivität der Mischung liegt.

2. Mischung von Superoxiddismutasen nach Anspruch 1, **dadurch gekennzeichnet, dass** die Mischung eine gesamte SOD-Aktivität von über oder gleich 130 U/mg der Mischung aufweist.

3. Mischung von Superoxiddismutasen nach Anspruch 1 oder 2, **dadurch gekennzeichnet, dass** die erste Isoform der Eisen-Superoxiddismutase ein Molekulargewicht von etwa 32.200 Da aufweist.

4. Mischung von Superoxiddismutasen nach einem der Ansprüche 1 bis 3, **dadurch gekennzeichnet, dass** die zweite Isoform der Eisen-Superoxiddismutase ein Molekulargewicht von etwa 79.800 Da aufweist.

5. Mischung von Superoxiddismutasen nach Anspruch 1, **dadurch gekennzeichnet, dass** die kumulierte SOD-Aktivität der beiden Isoformen der Eisen-Superoxiddismutasen zwischen 22% und 26% der gesamten SOD-Aktivität der Mischung liegt.

6. Mischung von Superoxiddismutasen nach einem der Ansprüche 1 bis 5, **dadurch gekennzeichnet, dass** die Mangan-Superoxiddismutase ein Molekulargewicht zwischen 70.000 und 90.000 Da aufweist und die Kupfer- und Zink-Superoxiddismutase ein Molekulargewicht zwischen 27.000 und 33.000 Da aufweist.

7. Mischung von Superoxiddismutasen nach einem der Ansprüche 1 bis 6, **dadurch gekennzeichnet, dass** sie durch Mahlen oder Pressen in wässrigem Medium, vorzugsweise bei einem pH-Wert von 5 bis 9, der Hybridvarietät F1 von Cucumis Melo MA 7950 oder ihrer in vitro kultivierten Zellen, dann Rückgewinnen des Überstands und Reinigen durch Chromatographie, insbesondere durch IMAC-Chromatographie, erhaltbar ist.

8. Nahrungs-, kosmetische oder pharmazeutische Zusammensetzung, die als wirksamen Inhaltsstoff eine Mischung aus Superoxiddismutasen nach einem der Ansprüche 1 bis 6 und mindestens einen für Nahrung, pharmazeutisch oder kosmetisch akzeptablen Trägerstoff enthält.

9. Kosmetische Zusammensetzung nach Anspruch 8, **dadurch gekennzeichnet, dass** sie für eine äußere topische Verwendung wie beispielsweise Pflegeprodukte, Shampoos, Lotionen, Gele bestimmt ist.

10. Nicht-therapeutische Verwendung einer Zusammensetzung, die als wirksamen Inhaltsstoff eine Mischung von Superoxiddismutasen nach einem der Ansprüche 1 bis 6 und mindestens einen akzeptablen Trägerstoff zur Verstärkung der Wirkung anderer verwendeter Antioxidantien enthält, die bei einem Tier verwendet werden.

11. Nicht-therapeutische Verwendung einer Zusammensetzung, die als wirksamen Inhaltsstoff eine Mischung von Superoxiddismutasen nach einem der Ansprüche 1 bis 6 und mindestens einen akzeptablen Trägerstoff zur Stimulierung der Vitalität der Zellen bei einem Tier enthält.

12. Pharmazeutische Zusammensetzung nach Anspruch 8, **dadurch gekennzeichnet, dass** sie für eine topische, orale, nasale oder parenterale Verabreichung bestimmt ist und beispielsweise in Form einer Tablette, einer Kapsel, einer Weichkapsel, einer Brausetablette, eines Beutels oder eines Sticks zum Verdünnen, eines Sirups, eines Elixiers, von Kräutertee, Kaugummi, eines Sprays, eines Aerosols oder einer injizierbaren Lösung vorliegt.

13. Pharmazeutische Zusammensetzung nach Anspruch 8 oder 12, **dadurch gekennzeichnet, dass** sie einen weiteren Wirkstoff, vorzugsweise ein weiteres Antioxidans, enthält.

14. Pharmazeutische Zusammensetzung nach einem der Ansprüche 8, 12 oder 13 zu ihrer Verwendung als Arzneimittel.

15. Pharmazeutische Zusammensetzung zur Verwendung nach Anspruch 14, **dadurch gekennzeichnet, dass** das Arzneimittel zur Behandlung oder Vorbeugung von Krankheiten im Zusammenhang mit oxidativem Stress und/oder einer Entzündung und/oder zur Behandlung der Mukoviszidose oder der Friedrich-Ataxie bei einem Tier bestimmt ist.

16. Pharmazeutische Zusammensetzung zur Verwendung nach Anspruch 15, **dadurch gekennzeichnet, dass** das Tier der Mensch ist.

17. Pharmazeutische Zusammensetzung zur Verwendung nach Anspruch 14, **dadurch gekennzeichnet, dass** das Arzneimittel zur Behandlung von Herz-Kreislauf-Erkrankungen, Fettleibigkeit, Arteriosklerose und Lippenherpes, insbesondere beim Menschen, bestimmt ist.

18. Nahrungszusammensetzung nach Anspruch 8, **dadurch gekennzeichnet, dass** sie zur oralen Verabreichung bestimmt ist und beispielsweise in Form von Tabletten, Kapseln, Weichkapseln, Brausetabletten, Beuteln oder Sticks zum Auflösen, Kaugummi, Getränken, Säften, Joghurt, Süßigkeiten, Keksen oder Riegeln vorliegt.

## Claims

1. A mixture of superoxide dismutases of plant origin, **characterized in that** it is essentially consisting of 3 superoxide dismutases: a manganese superoxide dismutase, a copper and zinc superoxide dismutase and an iron superoxide dismutase in at least two isoforms, the first isoform of iron superoxide dismutase having a molecular weight of between 28 000 and 36 000 Da, the second isoform of iron superoxide dismutase having a molecular weight of between 75 000 and 85 000 Da, said mixture able to be obtained from an extract of the F1 hybrid variety of *Cucumis Melo* MA 7950 or the cells thereof cultured *in vitro* or via transfer and expression of the genes of these SODs in prokaryote or eukaryote cells.

2. The mixture of superoxide dismutases according to claim 1, **characterized in that** said mixture has a total SOD activity equal to or higher than 130 U/mg of said mixture.

3. The mixture of superoxide dismutases according to claim 1 or 2, **characterized in that** the first isoform of iron superoxide dismutase has a molecular weight of about 32 200 Da.

4. The mixture of superoxide dismutases according to any one of claims 1 to 3, **characterized in that** the second isoform of iron superoxide dismutase has a molecular weight of about 79 800 Da.

5. The mixture of superoxide dismutases according to any one of claims 1, **characterized in that** the accumulated SOD activity of the two isoforms of iron superoxide dismutase is between 22 % and 26 % of the total SOD activity of the mixture.

6. The mixture of superoxide dismutases according to any one of claims 1 to 5, **characterized in that** the the manganese superoxide dismutase has a molecular weight of between 70 000 and 90 000 Da, and the copper and zinc superoxide dismutase has a molecular weight of between 27 000 and 33 000 Da.

7. The mixture of superoxide dismutases according to any one of claims 1 to 6, **characterized in that** it is able to be obtained by grinding or pressing in an aqueous medium, preferably at a pH of 5 to 9, the F1 hybrid variety of *Cucumis Melo* MA 7950 or the cells thereof cultured *in vitro* or via transfer and expression of the genes of these SODs in prokaryote or eukaryote cells, followed by recovery of the supernatant and purification by chromatography, IMAC chromatography in particular.

8. A nutritional, cosmetic or pharmaceutical composition containing as active ingredient a mixture of superoxide dismutases according to any one of claims 1 to 6 and at least one pharmaceutically or cosmetically acceptable food-grade excipient.

9. The cosmetic composition according to claim 8, **characterized in that** it is intended for external topical use such as care products, shampoos, lotions, gels.

10. Non-therapeutic use of a composition containing as an active ingredient a mixture of superoxide dismutases according to any of claims 1 to 6 and at least one acceptable excipient, to enhance the action of other antioxidants used in an animal.

11. Non-therapeutic use of a composition containing as an active ingredient a mixture of superoxide dismutases according to any of claims 1 to 6 and at least one acceptable excipient, for stimulating cell vitality in an animal.

12. The pharmaceutical composition according to claim 8, **characterized in that** it is intended for administration via topical, oral, nasal or parenteral route, and for example is in the form of a tablet, hard capsule, soft capsule, effervescent tablet, sachet or stick to be diluted, syrup, elixir, herbal tea, chewing gum, spray, aerosol or solution for injection.

13. The pharmaceutical composition according to claim 8 or 12, **characterized in that** it contains another active ingredient, advantageously another antioxidant.

14. The pharmaceutical composition according to any one of claims 8, 12 or 13 for use thereof as medicinal product.

15. Pharmaceutical composition for use according to claim 14, **characterized in that** the medicinal product is intended to treat or prevent diseases in animals related to oxidative stress and/or inflammation or to treat cystic fibrosis or Friedrich ataxia.

16. Pharmaceutical composition for use according to claim 15, **characterized in that** the animal is human being.

17. Pharmaceutical composition for use according to claim 14, **characterized in that** the medicinal product is intended for the treatment of cardiovascular diseases, obesity, arteriosclerosis and labial herpes, in particular in humans.

18. Nutritional composition according to claim 8, **characterized in that** it is intended for administration via oral route and for example is in the form of a tablet, hard capsule, soft capsule, effervescent tablet, sachet or stick to be diluted, chewing gum, beverages, juices, yoghurt, confectionery, biscuit or bars.
